Europäisches Patentamt

European Patent Office

Office européen des brevets

(11) Numéro de publication: **0 298 786**
**A1**

# DEMANDE DE BREVET EUROPEEN

(21) Numéro de dépôt: **88401310.3**

(22) Date de dépôt: **30.05.88**

(51) Int. Cl.⁴: **G 06 F 15/20**

(30) Priorité: **29.05.87 FR 8707600**

(43) Date de publication de la demande:
**11.01.89 Bulletin 89/02**

(84) Etats contractants désignés:
**DE FR GB IT NL SE**

(71) Demandeur: **CENTRE NATIONAL DE LA RECHERCHE SCIENTIFIQUE (CNRS)**
**15, Quai Anatole France**
**F-75700 Paris Cedex 07 (FR)**

(72) Inventeur: **Lumeau, Bernard Jean-François**
**1, Allée du Lot**
**F-31770 Colomiers (FR)**

**Clergeot, Henri**
**4 Allée de Québec**
**F-91300 Massy (FR)**

(74) Mandataire: **Joly, Jean-Jacques et al**
**CABINET BEAU DE LOMENIE 55, rue d'Amsterdam**
**F-75008 Paris (FR)**

Le titre de l'invention a été modifié (Directives relatives à l'examen pratiqué à l'OEB, A-III, 7.3)

(54) **Dispositif d'analyse spectrale mono et multidimensionnelle optimisant le compromis biais-variance.**

(57) Les signaux provenant de sources (10) sont convertis sous forme numérique (14) et subissent une transformation de Fourier pour obtenir leurs transformées fréquentielles. Le circuit de traitement (20) est agencé pour calculer le périodogramme d'un signal, l'interpériodogramme de deux signaux et estimer une matrice de densité interspectrale. Des moyens (24) réalisent le lissage optimal du Log-périodogramme ou de l'interpériodogramme de deux signaux blanchis par calcul d'une fenêtre de pondération optimale. Les autospectres et interspectres estimés sont obtenus après lissage. Par mise en oeuvre d'un algorithme récursif comprenant des estimations d'autospectres et d'interspectres, un ensemble de signaux peut être décorrélé et blanchi, conduisant à l'estimation de la matrice interspectrale. L'invention trouve une application particulière dans le traitement de signaux électroencéphalographiques, notamment pour la cartographie cérébrale, la localisation de sources d'activité cérébrale et la recherche de relations entre celles-ci.

Fig.1

**Description**

**Dispositif d'analyse spectrale pour l'estimation optimale de l'autospectre d'un signal de l'interspectre de deux signaux et son application à l'estimation d'une matrice interspectrale.**

La présente invention concerne un dispositif d'analyse spectrale pour l'estimation optimale de l'autospectre d'un signal, de l'interspectre de deux signaux et son application à l'estimation d'une matrice interspectrale sous forme factorisée.

Un domaine particulier de l'invention est celui du traitement de signaux physiologiques, notamment de signaux électroencéphalographiques (signaux EEG).

Dans ce domaine particulier, il existe déjà des dispositifs destinés à réaliser une cartographie cérébrale à partir d'autospectres de signaux EEG recueillis en différentes parties du scalp. La cartographie cérébrale consiste à élaborer des cartes illustrant par des nuances de gris ou par des couleurs différentes la répartition spatiale de la puissance d'émission à différentes fréquences.

Une utilisation particulière de la cartographie consiste dans la recherche de foyers épileptiques. Il est alors nécessaire de disposer de dispositifs d'analyse spectrale permettant d'estimer de façon fiable des autospectres de signaux EEG afin d'effectuer un diagnostic sûr.

Or, une difficulté majeure du traitement de signaux physiologiques tient au fait que ces signaux sont généralement noyés dans un bruit important. Un procédé classique utilisé en analyse spectrale consiste dans le lissage du périodogramme, ce qui implique le choix d'une fenêtre de lissage. Avec les dispositifs existants, dans lesquels le choix de la fenêtre de lissage est laissé à l'opérateur, l'obtention de résultats fiables ne peut être garanti, d'où l'impossibilité de fonder un diagnostic de façon sûre. En effet, plus la fenêtre de pondération est large et plus l'effet de lissage est important : la variance est réduite, mais les variations rapides du spectre sont perdues. A l'inverse, une fenêtre de pondération étroite laisse subsister une variance mais diminue le biais d'estimation, c'est-à-dire l'écart entre valeur moyenne de l'estimée et valeur réelle.

C'est pourquoi un premier objet de la présente invention est de fournir un dispositif d'analyse spectrale grâce auquel le compromis biais-variance est automatiquement optimisé, afin d'estimer un autospectre de façon aussi précise que possible.

Mais, dans le domaine du traitement de signaux EEG, il est également utile de rechercher des cohérences entre signaux recueillis sur différentes électrodes pour rechercher des relations entre différentes sources d'activité cérébrale. Cette recherche peut être effectuée par une estimation des interspectres entre signaux EEG.

Aussi, un deuxième objet de la présente invention est de fournir un dispositif d'analyse spectrale permettant une estimation de l'interspectre de deux signaux assurant également une optimisation automatique du compromis biais-variance.

Plus généralement encore, l'estimation des éléments de la matrice interspectrale peut être un outil efficace pour réaliser une localisation spatiale précise des sources d'activité cérébrales et évaluer leurs relations éventuelles. Une optimisation séparée élément par élément faisant appel â des estimations d'autospectres et d'interspectres ne conduit pas à de bons résultats. En particulier, elle ne garantit pas le caractère défini positif de la matrice estimée.

Aussi, un troisième objet de la présente invention est de fournir un dispositif d'analyse spectrale faisant appel de façon récursive aux dispositifs ci-dessus d'estimation d'autospectres et d'interspectres et permettant une estimation d'une matrice interspectrale sous forme factorisée.

Les dispositifs d'analyse spectrale selon l'invention comprennent :
- des moyens de réception de signaux à analyser,
- des moyens convertisseurs pour convertir les signaux sous forme numérique, et
- des moyens de transformation de Fourier pour calculer les transformées fréquentielles des signaux numérisés.

Selon un premier aspect de l'invention, le dispositif d'analyse spectrale pour l'estimation d'un autospectre à partir des signaux fréquentiels comprend :
- des moyens de calcul du périodogramme et de transformation logarithmique pour transformer le périodogramme en Log-périodogramme,
- des moyens de lissage optimal, appliqués au Log-périodogramme, et
- des moyens de transformation logarithmique inverse pour obtenir l'autospectre estimé à partir du Log-périodogramme lissé.

Le périodogramme du signal recueilli peut être interprété comme le spectre à estimer noyé dans un bruit multiplicatif. Le passage au Log-périodogramme permet de se ramener au cas d'un signal à estimer perturbé par un bruit additif. Comme on le verra plus en détail par la suite, il est alors possible d'estimer de façon automatique la fenêtre de lissage optimale donnant le meilleur compromis biais-variance.

De préférence, des moyens sont prévus pour effectuer un prémoyennage sur le périodogramme avant le passage au Log-périodogramme, afin d'éviter, pour une fenêtre donnée, l'effet d'augmentation de la variance qui résulte du lissage du Log-périodogramme, comparé au lissage du périodogramme.

Selon un autre de ses aspects, l'invention propose un dispositif d'analyse spectrale, faisant notamment application du dispositif défini ci-dessus d'estimation de l'autospectre d'un signal, et destiné à estimer la cohérence et l'interspectre de deux signaux dispositif comprenant, conformément à l'invention :
- les moyens définis ci-dessus d'estimation d'autospectrès pour fournir les autospectres des deux signaux,
- des moyens de blanchiment des deux signaux et de calcul de l'interpériodogramme de ces derniers,

- des moyens de lissage optimal, appliqués à l'interpériodogramme pour obtenir une estimée de la cohérence, et

- des moyens de calcul de l'interspectre estimé à partir des autospectres et de la cohérence.

De préférence, des moyens sont en outre prévus pour effectuer un prémoyennage de l'interpériodogramme, avant lissage.

Les moyens de lissage optimal du Log-périodogramme ou de l'interpériodogramme comprennent des moyens de transformation de Fourier inverse pour transformer le Log-périodogramme ou l'interpériodogramme et obtenir une fonction temporelle, des moyens de calcul automatique d'une fenêtre de pondération optimale de la fonction temporelle, des moyens d'apodisation de la fonction temporelle par la fenêtre calculée et des moyens de transformation de Fourier directe pour transformer la fonction temporelle apodisée et obtenir le Log-périodogramme ou l'interpériodogramme lissé.

Selon encore un autre de ses aspects, l'invention propose un dispositif d'analyse spectrale faisant application des dispositifs définis ci-dessus d'estimation d'autospectres et de cohérences, pour estimer une matrice interspectrale, dispositif comprenant :

- des moyens de traitement pour transformer par combinaisons linéaires K signaux fréquentiels d'entrée à analyser en k signaux de sortie blanchis et décorrélés, et

- des moyens de calcul de la matrice M, correspondant à la transformation linéaire effectuée par les moyens de traitement, et de la matrice interspectrale sous forme du produit $MM^+$,

- les moyens de traitement utilisant les moyens définis ci-dessus d'estimation d'autospectres et de cohérences pour

(a) fournir le premier signal de sortie par estimation de l'autospectre du premier signal d'entrée et blanchiment de celui-ci,

(b) estimer la cohérence entre le premier signal de sortie et le deuxième signal d'entrée pour calculer la fraction de celui-ci corrélée avec le précédent, évaluer par différence la fraction du deuxième signal d'entrée décorrélée, et fournir le deuxième signal de sortie décorrélé du premier et blanc par estimation de l'autospectre de la fraction décorrélée du deuxième signal d'entrée et blanchiment de celui-ci, et

(c) successivement, pour chaque $i^{eme}$ signal d'entrée suivant, estimer la cohérence entre le $i^{eme}$ signal d'entrée et chacun des premier au $(i-1)^{eme}$ signaux de sortie pour calculer la fraction du $i^{eme}$ signal d'entrée corrélée avec les $(i-1)$ premiers signaux de sortie, évaluer par différence la fraction du $i^{eme}$ signal d'entrée décorrélée, et fournir le $i^{eme}$ signal de sortie décorrélé des précédents et blanc par estimation de l'autospectre de la fraction décorrélée du $i^{eme}$ signal d'entrée et blanchiment de celle-ci.

Un tel dispositif d'analyse conforme à l'invention présente plusieurs avantages.

D'abord, en utilisant, à chaque étape de la récursion, pour l'estimation des autospectres et des cohérences, des moyens de calcul de fenêtres de pondération optimales, on optimise l'estimation des éléments de la matrice M de blanchiment et de décorrélation.

De plus, le processus mis en oeuvre par le dispositif d'analyse garantit l'aboutissement à une matrice interspectrale sous forme factorisée nécessairement définie non négative.

L'invention sera mieux comprise à la lecture de la description faite ci-après, à titre indicatif mais non limitatif, en référence aux dessins annexés sur lesquels :

- la figure 1 illustre très schématiquement un mode de réalisation d'un dispositif d'analyse spectrale conforme à l'invention pour l'estimation d'autospectre, d'interspectre ou de matrice interspectrale ;

- les figures 2A et 2B sont des organigrammes montrant les phases successives mises en oeuvre par le dispositif d'analyse spectrale pour, respectivement, estimer un autospectre et calculer la fenêtre de lissage optimale ;

- les figures 3 et 4 sont des courbes illustrant la recherche de fenêtre de pondération optimale ;

- les figures 5A à 5D sont des courbes illustrant respectivement : le périodogramme d'un signal bruité dont l'autospectre est à estimer, l'autospectre obtenu au moyen du dispositif selon l'invention, et deux autospectres obtenus au moyen de dispositifs de l'art antérieur avec choix arbitraire de la largeur de la fenêtre de lissage ;

- la figure 6 est un organigramme montrant les phases successives pour estimer la cohérence entre deux signaux ;

- la figure 7 illustre l'algorithme mis en oeuvre pour estimer une matrice interspectrale.

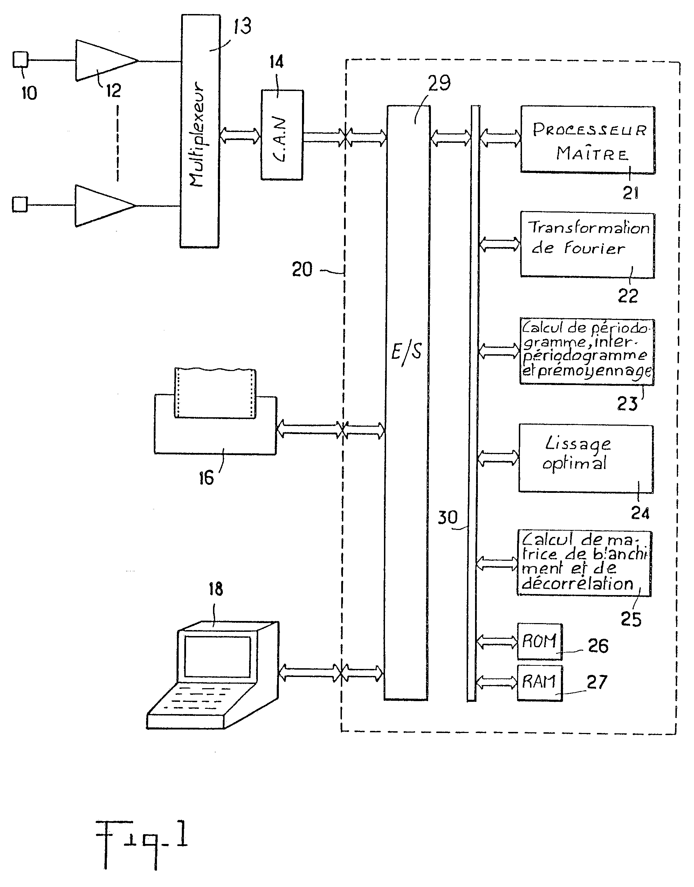

Les signaux à analyser, par exemple des signaux EEG recueillis au moyen d'électrodes de scalp 10 (Fig. 1) sont amplifiés au moyen d'amplificateurs 12 puis multiplexés par un multiplexeur 13 et convertis sous forme numérique au moyen d'un convertisseur analogique-numérique 14. Les signaux numérisés sont traités au moyen d'une unité de traitement 20.

Compte tenu du volume de traitement à effectuer, on utilise de préférence une unité de traitement 20 à architecture multiprocesseur, organisée autour d'un bus commun 30 et comprenant : un processeur maître assurant le séquencement des opérations et le calcul final de la matrice interspectrale ; un transformateur de Fourier rapide 22 ; un processeur 23 effectuant le calcul de périodogramme et d'interpériodogramme, les prémoyennages, et les transformations logarithmiques directes et inverses ; un processeur 24 effectuant l'opération de lissage optimal un processeur 25 effectuant le calcul de matrice de blanchiment et de décorrélation ; et une mémoire commune comportant une partie de mémoire morte (ROM) 26 pour la mémorisation des programmes et une partie de mémoire vive (RAM) 27 pour les données. De façon classique, différents périphériques (non tous représentés) sont connectés à l'unité de traitement 20 par l'intermédiaire d'un circuit d'entrées/sorties 29, dont, notamment, une imprimante 16 et une console 18 permettant de fournir l'autospectre, la cohérence ou l'interspectre sous forme de courbes imprimées.

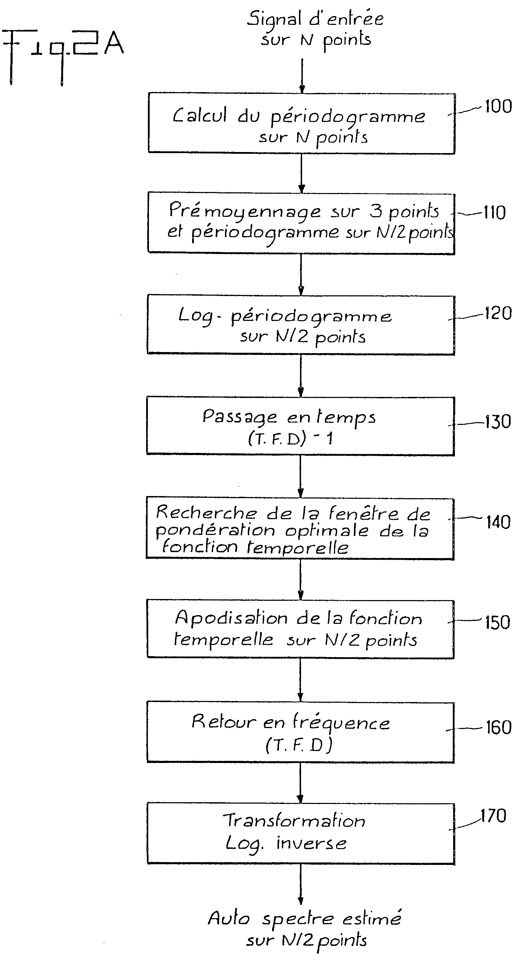

Les phases principales du programme d'estimation d'autospectre sont indequées sur la figure 2A.

La phase 100 consiste à calculer le périodogramme du signal d'entrée numérisé, c'est-à-dire à passer dans le domaine fréquentiel. Le calcul du périodogramme est effectué sur N points et est suivi d'un prémoyennage sur 3 points ce qui amène, par décalage d'un point, à un périodogramme sur N/2 points (phase 110).

Le prémoyennage sur trois points consiste à prendre pour chacun des N/2 points du périodogramme prémoyenné la moyenne arithmétique de trois points du périodogramme. L'estimation de l'autospectre étant effectué sur le Log-périodogramme, comme on le verra ci-après, le prémoyennage permet de compenser l'augmentation de variance qui résulte de la réalisation du lissage sur le Log-périodogramme plutôt que sur le périodogramme.

Le périodogramme prémoyenné est soumis à une transformation logarithmique pour obtenir un Log-périodogramme sur N/2 points (phase 120).

Le lissage du Log-périodogramme comprend les opérations successives de transformation de Fourier inverse (phase 130) pour passer dans le domaine temporel, de recherche de la fenêtre de pondération optimale de la fonction temporelle calculée sur N/2 points sur la transformée de Fourier inverse du Log-périodogramme (phase 140), d'apodisation de la fonction temporelle par la fenêtre optimale déterminée (phase 150) puis de retour en fréquence par transformée de Fourier directe (phase 160).

Le Log-périodogramme lissé ainsi obtenu est soumis à une transformation logarithmique inverse (phase 170) qui donne l'autospectre estimé sur N/2 points.

Le périodogramme du signal recueilli se présente comme le spectre à estimer noyé dans un bruit multiplicatif. Par le passage au Log-périodogramme, on se ramène à un spectre noyé dans un bruit additif et, selon une particularité du dispositif conforme à l'invention, il est alors possible d'estimer la fenêtre de pondération optimale pour l'opération de lissage, c'est-à-dire celle donnant le meilleur compromis biais-variance.

Le calcul de la fenêtre de pondération optimale (phase 140) comprend les phases principales indiquées sur la figure 2B.

A partir de la fonction temporelle calculée sur N/2 points (phase 130), on calcule (phase 142) la somme

$$S(I) = \sum_{l=1}^{I} SIG(l)^2 - 2\,RB \;,$$

dans laquelle :

- I représente la variable temps,
- I variant de 1 à N/2, est l'intervalle sur lequel a été calculé la somme,
- SIG (I) est la valeur de la fonction temporelle,
- RB est la valeur minimale de la variance du bruit lequel peut être assimilé à un bruit blanc stationnaire, RB = 1/3.

A partir de cette intégrale calculée, on recherche des intervalles significatifs de la fonction temporelle (phase 144) c'est-à-dire les intervalles pour lesquels la fonction /SIG(I)/$^2$ est en moyenne supérieure à 2RB, ce qui correspond aux intervalles où S(I) est en moyenne fonction croissante de I.

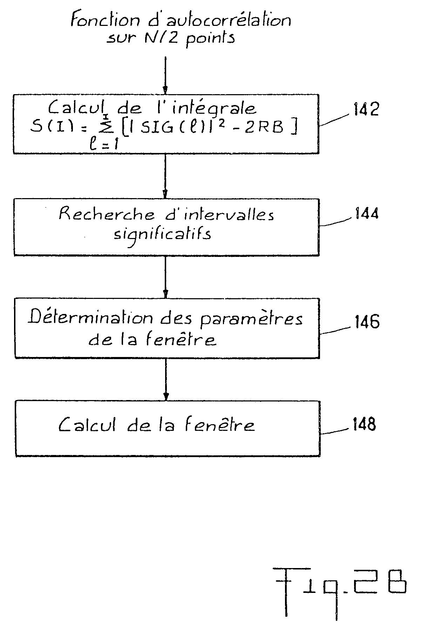

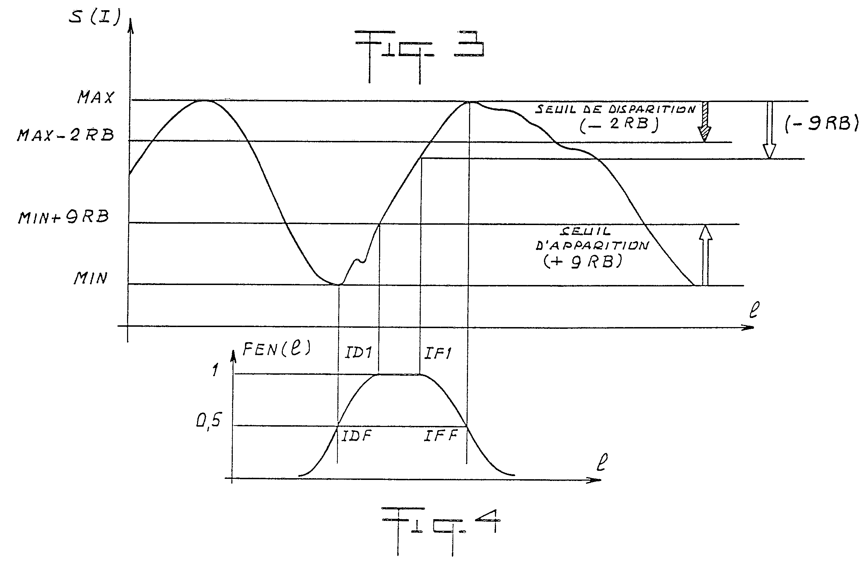

Les points caractéristiques (paramètres) de la fenêtre de pondération optimale sont déterminés par seuillage de l'intégrale calculée par rapport à la variance du bruit (phase 146). La figure 3 montre la variation de S(I). Le seuil d'apparition de signal est défini comme étant le seuil par rapport à un maximum ou à un minimum pour lequel la probabilité de franchissement en sens croissant en l'absence de signal est négligeable. Il est par exemple fixé à 9 RB en puissance (figure 3). Le seuil de disparition de signal est défini comme étant le seuil par rapport à un maximum ou un minimum pour lequel la probabilité de franchissement en sens décroissant en présence de signal est négligeable. Il est par exemple fixé à -2 RB en puissance (figure 3). Les bornes de la partie centrale des fenêtres de pondération (points ID1 et IF1 de la figure 4) sont celles correspondant au dépassement du seuil d'apparition de signal. Les autres points caractéristiques IDF et IFF de la fenêtre (figure 4) correspondent aux maxima et minima de S(I) et déterminent les points à mi-hauteur de la fenêtre de pondération.

Lorsque les paramétres des fenêtres optimales ont été déterminés, celles-ci sont calculées (phase 148) avant d'effectuer l'apodisation de la fonction temporelle (phase 150).

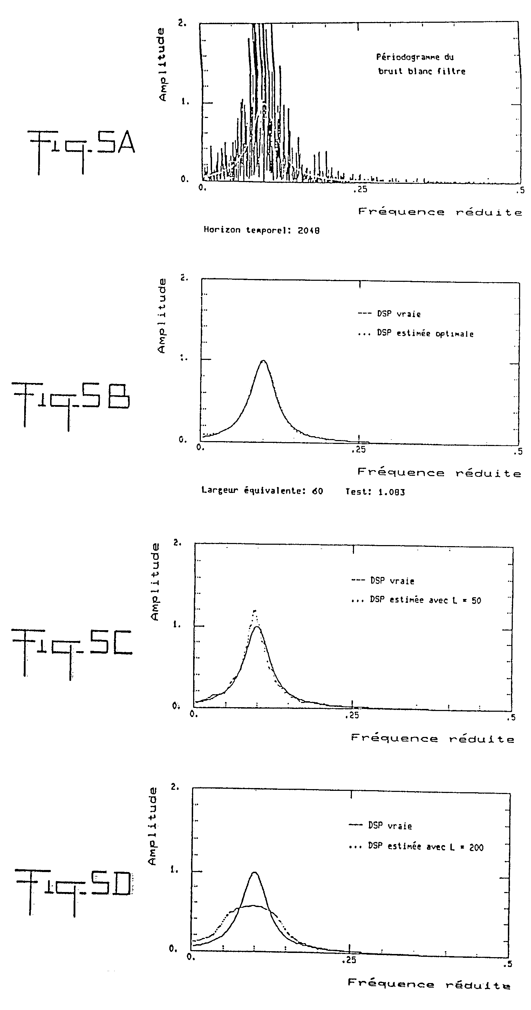

Les figures 5A à 5D montrent à titre d'exemple des résultats obtenus avec le dispositif conforme à l'invention et avec des dispositifs de l'art antérieur.

Sur la figure 5A, on a représenté le périodogramme d'un signal bruité dont l'autospectre est à estimer avec, en superposition l'autospectre (densité spectrale de puissance DSP) vrai.

La figure 5B montre, en trait plein la DSP vraie et en pointillés la DSP estimée optimale obtenue grâce au dispositif conforme à l'invention.

A titre de comparaison, les figures 5C et 5D montrent, en trait plein la DSP vraie et, en pointillés, les DSP estimées obtenues avec des dispositifs de l'art antérieur avec lesquels une largeur L de fenêtre de pondération doit être fixée arbitrairement, les valeurs de L étant respectivement 50 et 200 pour les figures 5C et 5D.

Les figures 5B à 5D montrent la précision que permet d'obtenir le dispositif selon l'invention dans l'estimation d'un autospectre. Un tel dispositif est donc particulièrement intéressant pour l'estimation d'autospectres de signaux EEG en ce sens qu'il permet de réaliser une cartograhie cérébrale sur la base de laquelle un diagnostic sûr peut être posé.

L'invention trouve également une application dans l'estimation de la cohérence entre deux signaux.

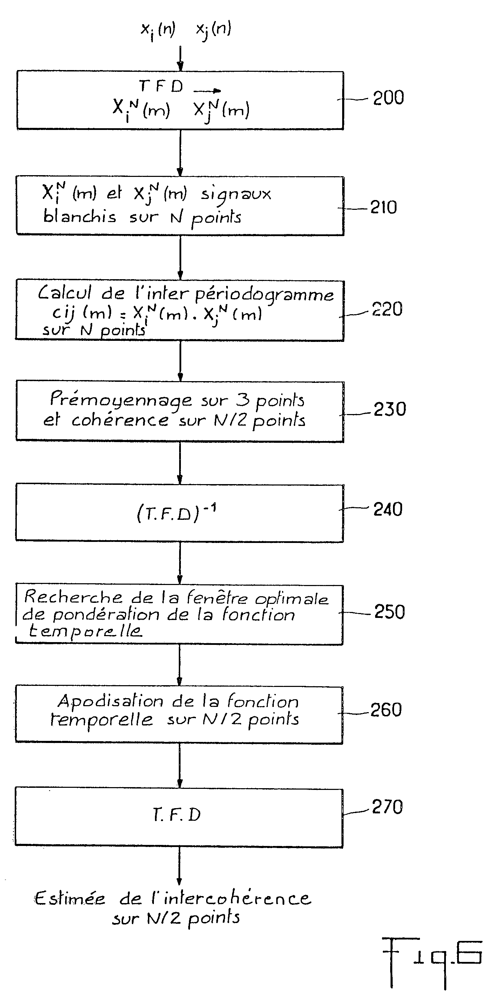

Les principales opérations d'estimation de la cohérence entre deux signaux (ou intercohérence de deux signaux) sont indiquées sur la figure 6.

Dans une phase 200, les deux signaux temporels $x_i(n)$ et $x_j(n)$ numérisés sur N points sont transformés par transformée de Fourier directe en signaux fréquentiels $X_i^N(m)$ et $X_j^N(m)$.

Les transformées de Fourier sont blanchies (phase 210). A cet effet, les autospectres en fréquence $\gamma_i^N(m)$ et $\gamma_j^N(m)$ des signaux $X_i^N(m)$ et $X_j^N(m)$ sont évalués pour calculer les transformées de Fourier blanchies.

$x_i^N(m) = X_i^N(m) \, \gamma_i^N(m)^{-1/2}$,
$x_j^N(m) = X_j^N(m) \, \gamma_j^N(m)^{-1/2}$.

L'estimation des autospectres est réalisée comme décrit ci-avant en référence aux figures 2A et 2B.

La cohérence $c_{ij}(m)$ entre les signaux est calculée sur N points (phase 220) en évaluant
$c_{ij}(m) = x_i^N(m) \cdot x_j^N(m)$.

Ensuite, l'interpériodogramme est prémoyenné sur trois points pour donner un interpériodogramme prémoyenné calculé sur N/2 points (phase 230).

Le lissage de l'interpériodogramme prémoyenné est effectué en passant dans le domaine temporel par transformation de Fourier inverse (phase 240), recherche de la fenêtre optimale de pondération de la fonction temporelle ainsi obtenue (phase 250), apodisation de la fonction temporelle par la fenêtre de pondération déterminée (phase 260) et retour au domaine fréquentiel par transformation de Fourier directe (phase 270).

La recherche de la fenêtre optimale de pondération est avantageusement réalisée d'une façon analogue à celle décrite plus haut en référence à la figure 2B.

La transformée de Fourier directe de la fonction temporelle apodisée constitue l'estimée recherchée de la cohérence, estimée obtenue sur N/2 points.

Un dispositif d'analyse spectrale pour estimer les éléments d'une matrice interspectrale peut être réalisé selon le schéma, déjà décrit, de la figure 1.

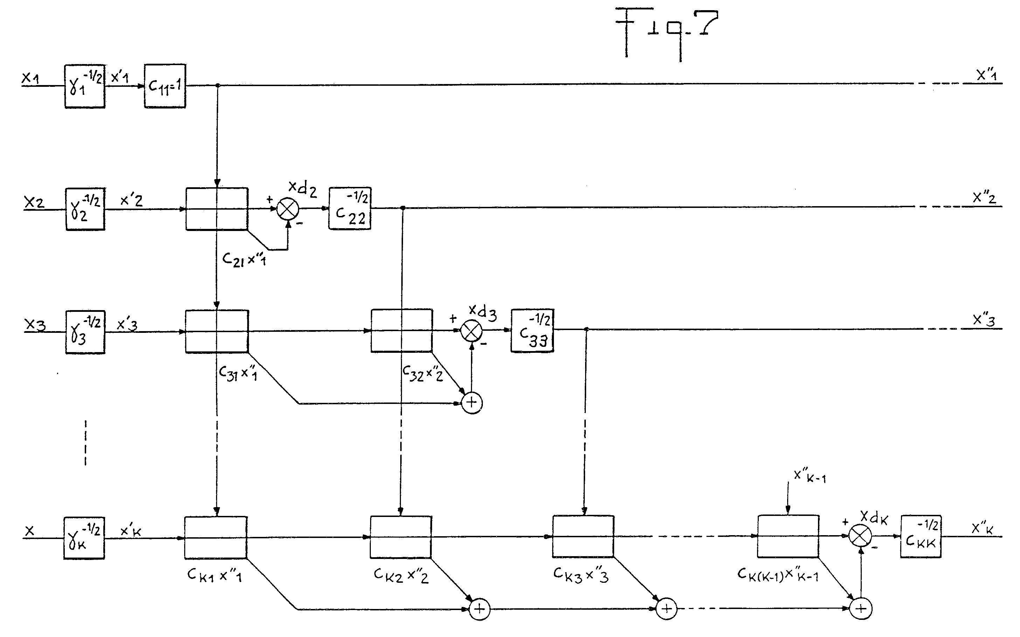

L'estimation des éléments de la matrice interspectrale est réalisée au moyen d'un programme stocké dans les circuits 26 et mettant en oeuvre notamment l'algorithme illustré par la figure 7.

Les signaux numérisés reçus $x_1$ à $x_K$ sont blanchis par estimations de leurs autospectres $\gamma_1$ à $\gamma_K$ et calcul de $x'_1 = x_1 \gamma_1^{-1/2}$ à $x'_K = x_K \gamma_K^{-1/2}$ (les indices de fréquence sont omis pour plus de clarté).

Le deuxième signal blanchi $x'_2$ est décorrélé du premier $x'_1$ par estimation de leur cohérence $c_{21}$ et calcul de la partie décorrelée $x_{d2} = x'_2 - c_{21} x'_1$. L'autospectre $c_{22}$ de la partie décorrelée est calculé, d'où l'on peut déduire un signal décorrélé blanchi $x''_2 = x_{d2} \cdot c_{22}^{-1/2}$.

De la même façon chaque nouveau signal blanchi $x'_i$ est décorrélé de chaque signal décorrélé blanchi précédent $x''_1$ à $x''_{i-1}$ par calcul des intercohérences $c_{i1}$ à $c_{i(i-1)}$ pour obtenir une partie décorrelée $x_{di} = x'_i - c_{i1} x''_1 - \cdots - c_{i(i-1)} x''_{i-1}$

Cette partie décorrelée $x_{di}$ est blanchie par estimation de l'autospectre $c_{ii}$ afin d'obtenir un signal décorrélé blanchi $x''_i = x_{di} \cdot c_{ii}^{-1/2}$.

Ainsi, la relation entre les signaux d'entrée $x_1$ à $x_K$ et les signaux décorrélés blanchis $x''_1$ à $x''_K$ fait apparaître un produit de deux matrices :
- une matrice diagonale dans laquelle les éléments sont les autospectres $\gamma_1^{1/2}$ à $\gamma_K^{1/2}$ des signaux $x_1$ à $x_K$ et
- une matrice triangulaire dans laquelle les éléments de la diagonale principale sont les autospectres $c_{11}^{1/2}$ à $c_{KK}^{1/2}$ des signaux décorrélés et les autres éléments, situés d'un côté de la diagonale, sont les cohérences $c_{12}$ à $c_{(K-1)K}$.

On assure ainsi à la matrice interspectrale le caractère défini non négatif nécessaire.

**Revendications**

1. Dispositif d'analyse comprenant des moyens de réception d'un signal à analyser, des moyens convertisseurs pour convertir le signal sous forme numérique, et des moyens de transformation de Fourier pour calculer la transformée fréquentielle du signal, caractérisé par :
- des moyens de traitement pour estimer l'autospectre du signal et comprenant des moyens de calcul du périodogramme du signal fréquentiel, des moyens de transformation logarithmique du périodogramme pour obtenir le Log-périodogramme, des moyens de lissage optimal du Log-périodogramme et des moyens de transformation logarithmique inverse du Log-périodogramme lissé pour obtenir l'autospectre estimé,
- les moyens de lissage du Log-périodogramme comprenant des moyens de transformation de Fourier inverse pour transformer le Log-périodogramme et obtenir une fonction temporelle, des moyens de calcul automatique d'une fenêtre de pondération optimale de la fonction temporelle, des moyens d'apodisation de la fonction temporelle par la fenêtre calculée et des moyens de transformation de Fourier directe pour transformer la fonction temporelle apodisée et obtenir le Log-périodogramme lissé.

2. Dispositif selon la revendication 1, caractérisé en ce qu'il comprend des moyens pour effectuer un prémoyennage du périodogramme avant le passage au Log-périodogramme.

3. Dispositif d'analyse spectrale comprenant des moyens de réception de signaux à analyser, des moyens convertisseurs pour convertir les signaux sous forme numérique et des moyens de transformation de Fourier pour calculer les transformées fréquentielles des signaux, caractérisé par des moyens pour estimer la cohérence de deux signaux et comprenant :
- des moyens d'estimation d'autospectres tels que définis dans l'une quelconque des revendications 1 et 2 pour calculer les autospectres des deux signaux,
- des moyens de blanchiment des deux signaux et de calcul de l'interpériodogramme de ces derniers, et

- des moyens de lissage optimal de l'interpériodogramme comportant des moyens de transformation de Fourier inverse pour transformer l'interpériodogramme et obtenir une fonction temporelle, des moyens de calcul automatique d'une fenêtre de pondération optimale de la fonction temporelle, des moyens d'apodisation de la fonction temporelle par la fenêtre calculée et des moyens de transformation de Fourier directe pour transformer la fonction temporelle apodisée et obtenir l'interpériodogramme lissé.

4. Dispositif selon la revendication 3, caractérisé en ce qu'il comprend en outre des moyens de calcul de l'interspectre des deux signaux à partir des autospectres et de la cohérence estimés.

5. Dispositif selon l'une quelconque des revendications 3 et 4, caractérisé en ce qu'il comprend en outre des moyens pour effectuer un pré-moyennage de l'interpériodogramme avant lissage.

6. Dispositif selon l'une quelconque des revendications 1 à 5, caractérisé en ce que les moyens de calcul automatique d'une fenêtre de pondération optimale comprennent :

- des moyens de calcul d'une intégrale de la fonction temporelle,

- des moyens de recherche des extrema de cette intégrale pour déterminer les points à mi-hauteur de la fenêtre de pondération, et

- des moyens de recherche des intervalles les plus significatifs de la fonction temporelle pour déterminer des points caractéristiques de la fenêtre de pondération.

7. Dispositif d'analyse spectrale pour estimer une matrice interspectrale, comprenant des moyens de réception de signaux temporels dont la matrice interspectrale est à estimer, des moyens convertisseurs pour convertir le signal sous forme numérique et des moyens de transformation de fourier pour calculer les transformées fréquentielles des signaux, dispositif caractérisé par :

- des moyens de traitement pour transformer par combinaisons linéaires K signaux fréquentiels d'entrée à analyser en K signaux de sortie blanchis et décorrélés, et

- des moyens de calcul de la matrice M, correspondant à la transformation linéaire effectuée par les moyens de traitement, et de la matrice interspectrale sous forme du produit $MM^+$,

- les moyens de traitement utilisant des moyens d'estimation d'autospectres tels que définis dans l'une quelconque des revendications 1 et 2, et des moyens d'estimation de cohérences tels que définis dans l'une quelconque des revendications 3 à 5 pour :

(a) fournir le premier signal de sortie par estimation de l'autospectre du premier signal d'entrée et blanchiment de celui-ci,

(b) estimer la cohérence entre le premier signal de sortie et le deuxième signal d'entrée pour calculer la fraction de celui-ci corrélée avec le précédent, évaluer par différence la fraction du deuxième signal d'entrée décorrélée, et fournir le deuxième signal de sortie décorrélé du premier et blanc par estimation de l'autospectre de la fraction décorrélée du deuxième signal d'entrée et blanchiment de celui-ci, et

(c) successivement, pour chaque $i^{ème}$ signal d'entrée suivant, estimer la cohérence entre le $i^{ème}$ signal d'entrée et chacun des premier au $(i-1)^{ème}$ signaux de sortie pour calculer la fraction du $i^{ème}$ signal d'entrée corrélées avec les $(i-1)$ premiers signaux de sortie, évaluer par différence la fraction du $i^{ème}$ signal d'entrée décorrélée, et fournir le $i^{ème}$ signal de sortie décorrélé des précédents et blanc par estimation de l'autospectre de la fraction décorrélée du $i^{ème}$ signal d'entrée et blanchiment de celle-ci.

8. Utilisation d'un dispositif d'analyse selon l'une quelconque des revendications précédentes pour le traitement de signaux électroencéphalographiques afin d'effectuer une cartographie cérébrale.

9. Utilisation d'un dispositif d'analyse selon l'une quelconque des revendications 1 à 7 pour le traitement de signaux électroencéphaliques afin d'effectuer une localisation spatiale de sources d'activité cérébrales.

0298786

Fig. 1

Fig.2A

Signal d'entrée
sur N points

| | |
|---|---|
| Calcul du périodogramme sur N points | —100 |

| | |
|---|---|
| Prémoyennage sur 3 points et périodogramme sur N/2 points | —110 |

| | |
|---|---|
| Log· périodogramme sur N/2 points | —120 |

| | |
|---|---|
| Passage en temps (T. F. D) $^{-1}$ | —130 |

| | |
|---|---|
| Recherche de la fenêtre de pondération optimale de la fonction temporelle | —140 |

| | |
|---|---|
| Apodisation de la fonction temporelle sur N/2 points | —150 |

| | |
|---|---|
| Retour en fréquence (T. F. D) | —160 |

| | |
|---|---|
| Transformation Log. inverse | —170 |

Auto spectre estimé
sur N/2 points

0298786

Fonction d'autocorrélation
sur N/2 points

Calcul de l'intégrale
$S(I) = \sum_{\ell=1}^{I} \left[ |SIG(\ell)|^2 - 2RB \right]$ — 142

Recherche d'intervalles
significatifs — 144

Détermination des paramètres
de la fenêtre — 146

Calcul de la fenêtre — 148

Fig.28

S(I)

Fig. 3

MAX
MAX-2RB
MIN+9RB
MIN

SEUIL DE DISPARITION
(-2RB)

(-9RB)

SEUIL
D'APPARITION
(+9RB)

ℓ

FEN(ℓ)

ID1     IF1

1

0,5

IDF     IFF

ℓ

Fig. 4

0298786

Fig.5A

Fig.5B

Fig.5C

Fig.5D

0298786

$$x_i(n) \quad x_j(n)$$

| $TFD \longrightarrow$ <br> $X_i^N(m) \quad X_j^N(m)$ | — 200 |

| $X_i^N(m)$ et $X_j^N(m)$ signaux <br> blanchis sur $N$ points | — 210 |

| Calcul de l'inter périodogramme <br> $cij(m) = X_i^N(m) . X_j^N(m)$ <br> sur $N$ points | — 220 |

| Prémoyennage sur 3 points <br> et cohérence sur $N/2$ points | — 230 |

| $(T.F.D)^{-1}$ | — 240 |

| Recherche de la fenêtre optimale <br> de pondération de la fonction <br> temporelle | — 250 |

| Apodisation de la fonction <br> temporelle sur $N/2$ points | — 260 |

| $T.F.D$ | — 270 |

Estimée de l'intercohérence
sur $N/2$ points

Fig.6

0298786

Fig.7

# RAPPORT DE RECHERCHE EUROPEENNE

Office européen des brevets

Numero de la demande

EP 88 40 1310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 155 670  (AMBOS)<br>* Page 7, ligne 35 - page 10, ligne 22; figures 4,5 *<br>--- | 1 | G 06 F  15/20 |
| A | ELECTRONIC DESIGN, vol. 33, no. 8, 4 avril 1985, pages 215-220,222, New Jersey, US; C. ERSKINE et al.: "Chip's hardware, software speeds up processing in laboratory instrumentation"<br>* Pages 215,216,219 *<br>--- | | |
| A | WO-A-8 601 391  (RAVIV)<br>--- | 1 | |
| A | IEEE INTERNATIONAL CONFERENCE ON ACOUSTICS, SPEECH AND SIGNAL PROCESSING, ICASSP'85 PROCEEDINGS, Florida, 1985, vol. 4, pages 1609-1612, IEEE; S. GANESAN et al.: "A multimicroprocessor system with distributed common memory for real-time digital correlation and spectrum analysis"<br>----- | | |

DOMAINES TECHNIQUES RECHERCHES (Int. Cl.4)

G 06 F  15/20

Le présent rapport a été établi pour toutes les revendications

| Lieu de la recherche | Date d'achèvement de la recherche | Examinateur |
|---|---|---|
| LA HAYE | 05-09-1988 | CHUGG D.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)